# EUROPEAN PATENT APPLICATION

(11) **EP 4 582 093 A1**
(43) Date of publication of application: **09.07.2025**
(21) Application number: 23860555.4
(22) Date of filing: 01.09.2023
(51) Int. Cl.: A61K 38/02, A61P 15/08

(54) **RUMINANT CONCEPTION CHANCE-IMPROVING COMPOSITION**

(30) Priority: 02.09.2022 US 202263403303 P
(71) Applicant: National University Corporation Hokkaido University, Hokkaido 060-0808 (JP)
(72) Inventor: KATAGIRI Seiji, Sapporo-shi, Hokkaido 060-0808 (JP); TAGAMI Takayoshi, Sapporo-shi, Hokkaido 060-0808 (JP); TANIDA Takashi, Sapporo-shi, Hokkaido 060-0808 (JP)
(74) Representative: Gevers & Orès
(86) International application number: PCT/JP2023/032160
(87) International publication number: WO 2024/048795

(57) **Abstract**

The present invention provides: a conception chance-improving composition for normalization of EGF concentration profile and conception chance improvement for a ruminant; and a method for improving ruminant conception chance using said composition. The present invention is: a ruminant conception chance-improving composition comprising, as an active ingredient, a peptide or polypeptide including the amino acid sequence represented by SEQ ID NO: 1; a ruminant conception chance-improving composition having, as an active ingredient, bovine osteopontin protein, a mutant of bovine osteopontin protein, or a partial peptide of these proteins, in which the protein, the mutant, and the partial peptide include the amino acid sequence represented by SEQ ID NO: 1; and a method for improving ruminant conception chance in which either of the conception chance-improving compositions is administered to the fornix vaginae, or the vicinity thereof, of the vaginal portion of a ruminant on an estrous day or the period immediately before/after such.

## Description

### Technical Field

The present invention relates to compositions for improving conception chance in ruminants such as cows.

This application claims priority to No. 63/403,303, filed in the United States on September 2, 2022, the contents of which are incorporated herein by reference.

### Background Art

A decrease in the conception chance of dairy cows is directly linked to a decrease in milk production, and is therefore one of the main causes of deteriorating farm management. Of the non-pregnant cows (low conception chance cows), despite the absence of any abnormalities in the findings of the genital examination, clinical symptoms, or abnormalities in the reproductive cycle, the cows that do not become pregnant despite repeated breeding are called repeat breeder (RB) cows. RB cows are causing significant economic losses in dairy management due to the significant delay in pregnancy due to repeated breeding. The biggest obstacle in countermeasures for RB cattle is that the cause of non-conception is not clear, and identifying the abnormalities that lead to non-conception will lead to countermeasures [sic.].

Many of the RB cows in dairy cows are believed to have a reduced uterine function, which is the place of embryonic development. For example, the expression level of epidermal growth factor (EGF), which is one of the endometrial growth factors, is one of the indicators for evaluating uterine function. Approximately 70% of RB cows have abnormal endometrial EGF concentration profiles. Specifically, an abnormality was observed in which the increase in concentration seen on days 2 to 4 and days 13 to 14 after estrus disappeared or decreased. Cows with endometrial EGF concentrations less than 4.7 ng/g of tissue weight on the third day after estrus have low conception rates. (Non-patent Literatures 1 and 2). By resolving this abnormality in the endometrial EGF concentration profile, conception chance is also improved (Non-patent Literature 2). EGF is thought to be directly linked to decreased conception chance because of its involvement in embryo development and the mechanism by which the mother recognizes the presence of the embryo. With the exception of countermeasures against bacterial infections of the uterus, which are common in cattle, resolution of the abnormal endometrial EGF concentration profile is currently the only method to improve conception chance in RB cattle that targets the uterus.

The therapeutic treatment, in combination with hormonal formulations, has been used as a method of normalizing abnormalities in the endometrial EGF concentration profile, but a new option with stable efficacy and available for the treatment of non-conception in cattle is expected. It has been empirically known that mating RB cows that do not become pregnant even after repeated artificial inseminations with bulls (natural mating) results in a high conception rate. Therefore, administering the protein contained in the seminal serum, which is a liquid component of the seminal fluid, to the RB cow normalized the EGF concentration in the endometrium, and also improved conception chance (Non-patent Literature 3). The effect was due to osteopontin (OPN) contained in seminal plasma (Non-patent Literatures 4). In addition, when OPN prepared from milk was administered to RB cows (non-patent literature 5), and when OPN recombinant protein was administered to RB cows, as with OPN derived from seminal serum, the endometrial EGF concentration profile was normalized, and conception chance improved.

### Prior art literatures

### Non-patent literatures

Non-Patent Literature 1: Katagiri and Takahashi, Theriogenology, 2004, vol. 62, p. 103-112.
Non-Patent Literature 2: Katagiri and Takahashi, Animal Reproduction Science, 2006, vol. 95, p. 54-66.
Non-Patent Literature 3: Badrakh et al., Journal of Reproduction and Development, 2020, vol. 66 (2), p. 149-154.
Non-Patent Literature 4: Badrakh et al., Japanese Journal of Veterinary Research, 2020, vol. 68 (2), p. 91-103.
Non-Patent Literature 5: Kyaw et al., Theriogenology, 2022, vol. 184, p. 26-33.

### Outline of the Invention

### Challenges that the invention seeks to solve

RB cows with an abnormal endometrial EGF concentration profile and who do not conceive after repeated breeding accounts for 5 to 6% of all dairy cows. In addition, approximately 25% of the cows showed an abnormal endometrial EGF concentration profile similar to that of the RB cows at 60 days after delivery, which is the time of the start of the next breeding after delivery. Postpartum cows also include cows other than dairy cows such as beef cattle. In addition, there are also female animals that has low conception chance and that do not become pregnant even after repeated breeding, regardless of the fact that there are no abnormalities in the findings of the reproductive organs test and no clinical symptoms or abnormalities in the reproductive cycle, even in livestock other than cattle. In response to these, we can expect to resolve economic loss, if the endometrial EGF concentration profile can be normalized to improve conception chance.

The present invention provides: a conception chance-improving composition for normalization of endometrial EGF concentration profile and improving the conception chance of ruminants such as cows; and a method for improving the conception chance of ruminants such as cows, using said composition.

### Means for Solving the Problem

As a result of intensive research to solve the above problems, the present inventors found that administering a peptide containing the integrin binding domain (SVAYGLK: SEQ ID NO: 1) in bovine OPN to RB cattle, similar to the recombinant protein of OPN, normalized the endometrial EGF concentration profile and improving conception chance; and administering a peptide containing the integrin-binding domain and the full-length bovine OPN protein also improved the conception chance of postpartum cows, thereby completing the present invention.

In other words, the present invention provides a ruminant conception chance-improving composition, a method for improving the conception chance according to ruminants, and a straw for artificial insemination.
[1] A ruminant conception chance-improving composition, which contains as an active ingredient a peptide or polypeptide containing the amino acid sequence represented by SEQ ID NO: 1 (SVAYGLK).
[2] The conception chance-improving composition according to [1] described previously, wherein the peptide or polypeptide comprises an amino acid sequence represented by SEQ ID NO: 2 (GRGDSVAYGLK).
[3] The conception chance-improving composition according to [1] or [2] described previously, wherein the peptide or polypeptide is a partial peptide of bovine osteopontin.
[4] A ruminant conception chance-improving composition, wherein the bovine osteopontin protein, a mutant of the bovine osteopontin protein, or a partial peptide of these proteins is the active ingredient, and wherein the protein, the mutant, and the partial peptide described previously comprise an amino acid sequence represented by SEQ ID NO: 1 (SVAYGLK).
[5] The conception chance-improving composition according to [4] described previously, wherein said protein, said mutant, and said partial peptide comprise an amino acid sequence represented by SEQ ID NO: 2 (GRGDSVAYGLK).
[6] The conception chance-improving composition according to [4] or [5] described previously, wherein the amino acid sequence of the mutant of the bovine osteopontin protein has an amino acid sequence of 90% or more sequence identity with the amino acid sequence of the bovine osteopontin protein.
[7] The conception chance-improving composition according to [4] described previously, wherein the peptide comprising the amino acid sequence represented by SEQ ID NOs: 1 or 2 is the active ingredient.
[8] The conception chance-improving composition according to any one of [1] to [7] described previously, wherein the ruminant is a post-partum ruminant.
[9] The conception chance-improving composition according to any of the above [1]-[8] described previously, wherein the ruminant is a cow, goat, sheep, deer, or giraffe.
[10] The conception chance-improving composition according to [8], wherein the ruminant is a cow that is 45 - 80 days after delivery.
[11] The conception chance-improving composition according to any of the above [1]-[9] described previously, wherein the ruminant is a cow with low conception chance.
[12] The conception chance-improving composition according to [11] described previously, wherein said cow with low conception chance is a cow whose increased in epidermal growth factor concentration in the endometrium, which is found in cow with normal conception chance on days 2-4 from estrus, has disappeared or is declining.
[13] The conception chance-improving composition according to [11] described previously, wherein the cow with low conception chance is a cow whose epidermal growth factor concentration in the endometrium on the third day after estrus is less than 4.7 ng/g of tissue weight.
[14] The conception chance-improving composition according to [11] above, the active ingredient comprises:
   a protein comprising an amino acid sequence represented by SEQ ID NO: 1 (SVAYGLK) as a mutant of a protein of bovine osteopontin;
   a partial peptide of a protein of bovine osteopontin comprising the amino acid sequence represented by SEQ ID NO: 1 (SVAYGLK), or
   a partial peptide of a mutant of a protein of bovine osteopontin comprising an amino acid sequence represented by SEQ ID NO: 1 (SVAYGLK);
   wherein said cow with low conception chance is a repeat breeder cow.
[15] The conception chance-improving composition according to any one of [1] to [14] described previously, wherein the administration of the conception chance-improving composition normalizes the epidermal growth factor concentration profile in the endometrium from administration to one cycle of estrus.
[16] A method of improving conception chance in a ruminant, wherein the conception chance-improving composition according to any of [1] to [15] described previously is administered to or in the vicinity of the fornix vaginae of the ruminant at the estrus date and before and after the estrus date.
[17] The method of improving conception chance according to [16] described previously, wherein it is a solution in which the active ingredient is dissolved; a solution in which 0.1 to 20 mL of the solution is dissolved; the solution that can be administered to ruminants;
   a solution that can be administered to the vagina, cervix, and uterus of ruminants; a solution that can be water, a buffer solution, the semen of the ruminant, or a solution containing appropriate and pharmaceutically acceptable additives that can be administered to the vagina, cervix, and uterus of the ruminant.
[18] The method of improving conception chance according to [16] or [17] described previously, wherein the amount of active ingredient contained in the conception chance-improving composition is 40 nmol or greater.
[19] The method for improving conception chance according to any one of [16] to [18] described previously, wherein the conception chance-improving composition is administered before, after, or simultaneously with artificial insemination.
[20] The method for improving conception chance according to any one of [16] to [18] described previously, wherein the ruminant is a ruminant that naturally mates.
[21] The method for improving conception chance according to any one of [16] to [18] described previously, wherein the ruminant is a ruminant into which a fertilized egg is implanted.
[22] The method for improving conception chance according to any one of [16] to [21] described previously, wherein the ruminant is a cow, goat, sheep, deer, or giraffe.
[23] A straw for artificial insemination, wherein the straw for artificial insemination is filled with the conception chance-improving composition according to any one of [1] to [15] described previously;
   wherein the active ingredient can be dissolved in 0.1 to 20 mL of the solution; the solution of which can be administered to ruminants,
   a solution that can be administered to the vagina, cervix, and uterus of ruminants; a solution that can be water, a buffer solution, the semen of the ruminant, or a solution containing appropriate and pharmaceutically acceptable additives that can be administered to the vagina, cervix, and uterus of the ruminant.

### Effect of the invention

According to the present invention, it is possible to normalize the endometrial EGF concentration profile and improve the conception chance of ruminants with abnormal endometrial EGF concentration profiles or postpartum ruminants.

### Brief Description of the Drawings

[FIG. 1] A schematic illustration of changes (solid lines) over time after estrus for the endometrial EGF concentration in RB cows.
[FIG. 2] A drawing showing the schedule for treatment with OPN in Reference Example 1.
[FIG. 3] A drawing showing the measurement results of endometrial EGF concentration on the third day after treatment of RB cows subjected to treatments with PBS, SP, and rOPN in Reference Example 1.
[FIG. 4] A schematic illustration of the structure of a partial peptide or partial protein of a treatment with OPN used in Example 1.
[FIG. 5] A drawing showing the measurement results of endometrial EGF concentration on the third day after treatment of RB cows, in which in Example 1, PBS treatment, rOPN treatment, peptide 1 treatment, peptide 2 treatment, peptide 3 treatment, and C-rOPN treatment were performed.
[FIG. 6] A drawing illustrating the schedule for treatment with OPN in Example 4.

### Best Mode For Carrying Out The Invention

Illustrative embodiments of the present invention are described in detail below.

In the present invention and the specification of the present application, "X₁ to X₂ (where X₁ and X₂ are real numbers that satisfy X₁ < X₂)" means "X₁ or greater and X₂ or less".

In the present invention and the specification of the present application, "polypeptide" refers to a polymer in which 20 or more amino acids are polymers bonded to peptide, and "peptide" refers to a polymer in which 2 to 19 amino acids are polymers bonded to peptide.

In the present invention and the specification of the present application, "osteopontin (OPN)" collectively refers to OPNs derived from various tissues of ruminants, unless otherwise stated. For example, "bovine osteopontin (OPN)" collectively refers to OPNs derived from various tissues of the cow (serum, milk, urine, blood, bone tissue, etc.). The OPNs include the various isoforms obtained by selective splicing, and also include post-translational modification of the OPNs. Post-translational modifications include phosphorylation, glycosylation, methylation, acetylation, nitrosylation, ubiquitination, and the like. OPNs also include recombinant proteins, not only natural (generated in the body of an animal) OPNs.

In the present invention and the specification of the present application, "the protein of osteopontin" refers to the full length of the mature protein of osteopontin. For example, it refers to a protein consisting of the 17-278th region of the amino acid sequence of SEQ ID NO: 4 (UniProt ID: P31096) of a bovine OPN. Of the amino acid sequences of SEQ ID NO: 4, the region 1 to 16 of the N-terminal is cleaved in the mature protein being secreted due to the signal sequence portion.

In the present invention and the specification of the present application, "osteopontin protein mutant" or "osteopontin mutant" refer to a protein in which one or more of the amino acids constituting a protein of osteopontin are replaced, deleted, or added, unless otherwise stated.

In the present invention and the specification of the present application, "amino acids are deleted in polypeptides (proteins) or peptides" refers to the loss (removal) of a portion of amino acid residues constituting a polypeptide or peptide.

In the present invention and the specification of the present application, "amino acids are substituted in a polypeptide (protein) or peptide" refers to the amino acid residues constituting the polypeptide or peptide are changed to another amino acid residues.

In the present invention and the specification of the present application, "amino acids are added in a polypeptide (protein) or peptide" refers to new amino acid residues are inserted into the polypeptide or peptide.

In the present invention and the specification of the present application, the term "estrus" of a ruminant refers to a physiological state in which a female ruminant can copulate, and normally, behaviors and signs specific to the ruminant that permit copulation are observed. For example, bovine estrus is repeated periodically at intervals of 18 to 24 days, and one estrus lasts for several hours to about a day.

In the present invention and the specification of the present application, "estrus date" of a ruminant refers to a date on which behaviors and signs specific to the ruminant that are observed during the estrus period are confirmed.

In the present invention and the specification of the present application, "endometrial EGF concentration profile abnormality" refers to an abnormality in which the periodic increase in the EGF concentration in the endometrium of the ruminant disappears or decreases. For example, in cows, it refers to an abnormality in which the increase in the concentration (appears as a peak on the concentration profile) observed on Days 2-4 and 13-14 after estrus in normal cows disappears or decreases. In normal cattle, the endometrial EGF concentrations of both peaks are generally within the range of 4.70 to 13.50 ng/g of tissue. In the present invention and the specification of the present application, the endometrial EGF concentration on the third day from the date of estrus or artificial insemination was measured, and the cow that has less than 4.70 ng/g of tissue was determined to have an abnormal endometrial EGF concentration profile.

In the present invention and the specification of the present application, "normalization of the endometrial EGF concentration profile" means that the periodic increase in the concentration of EGF in the endometrium seen in normal ruminants is confirmed in the ruminants that had an abnormal endometrial EGF concentration profile. In the case of cows, this refers to the periodic increases in the concentration of EGF, which are observed on days 2 to 4 and days 13 to 14 after estrus, observed in cows with abnormal endometrial EGF concentration profiles, similar to that of normal cows. It can be confirmed that the endometrial EGF concentration profile of the cow was normal, when the endometrial EGF concentration reached 4.70 ng/g of tissue or higher on the third day from the date of onset or artificial insemination of the cow with abnormal endometrial EGF concentration profile.

The endometrial EGF concentration profile of the ruminant is determined by collecting the endometrium of the ruminant over time in the estrus cycle and measuring the EGF amount in the tissue. The endometrium can be collected using a commonly used biopsy instrument, as described in Examples below. The amount of EGF in the endometrial tissue can be measured using the ELISA method, etc., as described in the non-patent literatures 1-4.

In the present invention and the specification of the present application, "a cow with low conception chance " refers to "a cow that has no detectable abnormalities in its reproductive organs or estrus cycle, but is difficult to become pregnant." and "RB cow" refers to "a cow that has no detectable abnormalities in its reproductive organs or estrus cycle, but has not become pregnant even after three or more inseminations." The diagnosis is usually made around 150 days after delivery.

### <Compound for improving conception chance >

The conception chance-improving composition of this embodiment contains a peptide or polypeptide comprising an integrin binding motif, the SVAYGLK motif (SEQ ID NO: 1). As shown in the example below, the SVAYGLK motif is a domain present in the OPN and is involved in resolving an endometrial EGF concentration profile abnormality by the OPN.

A peptide composed of the amino acid sequence represented by SEQ ID NO: 1 (a peptide consisting only of the SVAYGLK motif) can be an active ingredient in the conception chance-improving composition in this embodiment. Administration of the peptide to the vaginal portion of a ruminant provides effects of normalizing the EGF concentration profile of the endometrium and improving conception chance. That is, the SVAYGLK motif peptide has the ability to normalize the endometrial EGF concentration profile and improve the conception rate.

The substance, which is the active ingredients of the conception chance-improving composition of this embodiment, is not particularly limited as long as it has the SVAYGLK motif and has the ability to normalize the endometrial EGF concentration profile and improve conception chance. It is also possible to determine whether a certain substance has the ability to normalize the endometrial EGF concentration profile and improve the conception rate by performing the following procedure: Administering the substance into the vagina of ruminants with confirmed abnormal endometrial EGF concentration profile on the day of estrus (including one day before and after the day of estrus) or during artificial insemination, measuring the endometrial EGF concentration at a time corresponding to the period when normal animals show a periodic increase in endometrial EGF (for cows, the third day from the date of estrus or artificial insemination); and checking the conception rate. If the endometrial EGF concentration on the third day from the date of estrus or artificial insemination is 4.70 ng/g of tissue or higher, the substance is judged to have the ability to normalize the endometrial EGF concentration profile.

The active ingredient of the conception chance-improving composition in this embodiment is not particularly limited, as long as it contains the amino acid sequence represented by SEQ ID NO: 1; It may be a peptide or a polypeptide (protein).

The active ingredient of the conception chance-improving composition in this embodiment includes, e.g., a partial protein (which may be a partial peptide) of an OPN, which comprises an SVAYGLK motif. Specifically mentioned can be a peptide or polypeptide consisting of a partial region comprising the SVAYGLK motif (the 155-161 region of the amino acid sequence of SEQ ID NO: 4) in the amino acid sequence represented by SEQ ID NO: 4.

Especially preferred as an active ingredient of the conception chance-improving composition in this embodiment are peptides or polypeptides consisting of a partial region comprising both an RGD motif (152-154 region of the amino acid sequence of SEQ ID NO: 4) and an SVAYGLK motif in the OPN. Having both integrin-binding motifs in the OPN allows for better normalization of the endometrial EGF concentration profile and conception chance improvement than peptides, etc. containing only the SVAYGLK motif. Such peptides include peptides or polypeptides comprising an amino acid sequence represented by SEQ ID NO: 2 (GRGDSVAYGLK).

The active ingredient of the conception chance-improving composition in this embodiment may be a partial protein (which may be a partial peptide) of a mutant of OPN, comprising a SVAYGLK motif, and having the ability to normalize endometrial EGF concentration profile and the capability to improve conception chance. Such OPN mutants are not particularly limited, as long as it does not impair the ability of the SVAYGLK motif to normalize the endometrial EGF concentration profile and improve conception rate. Mutants of the OPN include, for example, a protein comprising an amino acid sequence having an sequence identity of 80% or more, preferably 90% or more but less than 100%, more preferably 95% or more less than 100%, and even more preferably 98% or less than 100% with full-length amino acid sequence of OPN. The active ingredient of the conception chance-improving composition of this embodiment includes a partial protein (or it may be a partial peptide) of a protein consisting of amino acid sequence represented by SEQ ID NO: 4 and consisting of an amino acid sequence having a sequence identity of 80% or more, preferably 90% or more and less than 100%, more preferably 95% or more and less than 100%, and still more preferably 98% or more and less than 100%. Those containing the SVAYGLK motif and having the ability to normalize the endometrial EGF concentration profile and improve conception rate are preferred. The active ingredient of the conception chance-improving composition of this embodiment includes a partial protein (or it may be a partial peptide) of a protein consisting of amino acid sequence represented by SEQ ID NO: 4 and consisting of an amino acid sequence having a sequence identity of 80% or more, preferably 90% or more and less than 100%, more preferably 95% or more and less than 100%, and still more preferably 98% or more and less than 100%. Preferably, it contains the amino acid sequence represented by SEQ ID NO: 2 (GRGDSVAYGLK) and has the ability to normalize the endometrial EGF concentration profile and improve conception chance.

The active ingredient of the conception chance-improving composition of this embodiment includes a partial protein (or it may be a partial peptide) of a protein consisting of amino acid sequence represented by SEQ ID NO: 4 and consisting of an amino acid sequence having a sequence identity of 80% or more, preferably 90% or more and less than 100%, more preferably 95% or more and less than 100%, and still more preferably 98% or more and less than 100%. Those containing the SVAYGLK motif and having at least one of the ability to normalize the endometrial EGF concentration profile and improve conception rate are preferred. The active ingredient of the conception chance-improving composition of this embodiment includes a partial protein (or it may be a partial peptide) of a protein consisting of amino acid sequence represented by SEQ ID NO: 4 and consisting of an amino acid sequence having a sequence identity of 80% or more, preferably 90% or more and less than 100%, more preferably 95% or more and less than 100%, and still more preferably 98% or more and less than 100%. Preferably, it contains the amino acid sequence represented by SEQ ID NO: 2 (GRGDSVAYGLK) and has the ability to normalize the endometrial EGF concentration profile and improve conception chance.

The active ingredient of the conception chance-improving composition in this embodiment may be a protein of an OPN. Also, the active ingredient of the conception chance-improving composition in this embodiment may be a mutant of a protein of OPN, comprising an SVAYGLK motif, preferably an amino acid sequence represented by SEQ ID NO:2 (GRGDSVAYGLK) and has the ability to normalize the endometrial EGF concentration profile and improve the conception rate.

Sequence identity (homology) between amino acid sequences is calculated by aligning the two amino acid sequences with gaps at the sites of insertion and deletion so that the number of corresponding amino acid residues is maximized, and then calculating the ratio of matching amino acid residues to the total amino acid sequence excluding gaps in the resulting alignment. Sequence identity between amino acid sequences can be determined using various homology search software known in the art. For example, the sequence identity value of an amino acid sequence can be obtained by calculation based on an alignment obtained using the known homology search software BLASTP.

The peptide or polypeptide that is the active ingredient of the conception chance-improving composition in this embodiment may have other amino acid sequence moiety, in addition to those derived from the partial region of the OPN, such as the peptide comprised of the SVAYGLK motif and the amino acid sequence represented by SEQ ID NO: 2 (GRGDSVAYGLK). The other amino acid sequence moieties may be attached to the N-terminal side of the portion derived from the partial region of the OPN, or to the C-terminal side [sic. "of the portion derived from the partial region of the OPN"].

Other amino acid sequence moieties include, for example, various signal peptides, various tag peptides, etc. These signal peptides and tag peptides can be appropriately selected from among various signal peptides and tag peptides commonly used in the manufacture of recombinant proteins. Such tag peptides include, for example, His tag, HA (hemagglutinin) tag, Myc tag, and Flag tag, etc.

In general, short-chain synthetic peptides have the problem of being easily degraded and having low stability. In addition, the charge to the N-terminus and C-terminus during the manufacturing process of the synthetic peptide may affect the function of the peptide itself. In order to solve such a problem, in the case of the active ingredient of the conception chance-improving composition of this embodiment is a fragment peptide of OPN (for example, a peptide consisting of the amino acid sequence represented by SEQ ID NO: 1 or 2), various measures known in the art can be taken, such as cyclization, modification of amino acid residues, substitution to specular (optic) isomers, etc.

By the cyclization of peptides, it is possible to expect the imitation of natural peptides and high stability in vivo. It also increases resistance to hydrolysis.

Alternatively, modification of the N-terminal and C-terminal amino acid residues is also preferred. By removing the charge of the N-terminus and C-terminus of the synthetic peptide, it is possible to have properties equivalent to those of the natural peptide. Therefore, when the active ingredient of the conception chance-improving composition of the present embodiment is a synthetic peptide, the charge at the terminal can be removed by acetylating the N-terminus or amidizing the C-terminus. These modifications also make degradation by peptidases difficult and improve stability.

All amino acids contained in natural peptides are L-forms, but by replacing them with their specular isomers (D-isomers), the function of the peptide can be modified. Even when the active ingredient of the conception chance-improving composition according to this embodiment is a synthetic peptide, improvement in stability, etc. can be expected by using some or all amino acids as D-amino acids. In addition, some or all of the amino acids constituting the synthetic peptide, which is the active ingredient, can be replaced with artificial amino acids.

When the active ingredient of the conception chance-improving composition of the present embodiment is a peptide, it can be easily synthesized using a commonly used peptide synthesis technique and is also economically efficient. Furthermore, when the active ingredient of the conception chance-improving composition of the present embodiment is a protein, it can be easily produced by using a widely used expression system such as [when producing] E. coli.

The conception chance-improving composition in this embodiment may be a composition consisting solely of an active ingredient and may be formulated by adding various additives to the active ingredient. The followings can be used as additives: General-purpose additives such as excipients, binders, lubricants, disintegrants, flow agents, solvents, solubilizers, buffers, suspending agents, emulsifiers, tonicity agents, stabilizers, preservatives, antioxidants, flavoring agents, and colorants. The dosage form of the conception chance-improving composition in this embodiment is not particularly limited, and may be a solid formulation such as powder, granules, tablets, etc., or may be a liquid formulation. The liquid preparation also includes a frozen one.

In addition to the active ingredient, or the active ingredient and additive[s], the conception chance-improving composition in this embodiment may contain a seminal content of a ruminant to which the conception chance-improving composition is administered. The semen component of a ruminant may be semen itself or a fraction such as seminal plasma. For example, a mixture in which the active ingredient is mixed with semen used for artificial insemination is also included in the conception chance-improving composition of the present embodiment.

When the conception chance-improving composition of this embodiment is administered to a ruminant having an abnormal endometrial EGF concentration profile, the epidermal growth factor concentration profile in the endometrium is normalized after administration for a period of one cycle of estrus. In the case of cows, the period for one cycle of estrus varies depending on the variety of cows, but it is approximately 20 to 30 days, and most are 21 days.

### <Method for improving conception chance in ruminants>

The method of improving conception chance in a ruminant of the present embodiment is a method of administering the conception chance-improving composition in the present embodiment to or in the vicinity of the vaginal fornix of the ruminant on the day of estrus and the period before and after it. Directly administering the conception chance-improving composition of the present embodiment to the vaginal fornix or its vicinity in the vagina of a ruminant can efficiently exert its effect on the endometrium.

The conception chance-improving composition of the present embodiment is administered to a ruminant on the day of estrus and the period before and after the same. By administering the conception chance-improving composition of the present embodiment before and after estrus, the effects of normalizing the abnormal endometrial EGF concentration profile and improving the conception rate are more fully exhibited. The administration of the conception chance-improving composition of the present embodiment is performed, for example, within a period of 5 days, preferably 4 days, more preferably 3 days, even more preferably 2 days after the date of estrus, from 5 days before the estrous date, preferably 4 days before, more preferably 3 days before, even more preferably 2 days before. It is particularly preferred that the administration of the conception chance-improving composition of this embodiment be performed within the period from one day prior to the estrus day and up to one day after the estrus day.

The ruminant to which the conception chance-improving composition according to this embodiment is administered may be a ruminant whose estrus or ovulation is artificially induced by hormone administration, etc., or may be a ruminant that has a natural estrus cycle. Administration to ruminants that have a natural estrus cycle also improves the conception rate of natural breeding.

The conception chance-improving composition of this embodiment is preferably applied to ruminants that are artificially inseminated. In the case of artificial insemination, administration of the conception chance-improving composition of this embodiment may be performed before, after, and at the same time as the artificial insemination. The administration of the conception chance-improving composition in this embodiment is performed, for example, within a period of up to 5 days, preferably 4 days, more preferably 3 days, even more preferably 2 days, even more preferably 1 day after artificial insemination, starting from 5 days before artificial insemination, preferably 4 days before, more preferably 3 days before, even more preferably 2 days before, even more preferably 1 day before. In the case of artificial insemination, administration of the conception chance-improving composition of this embodiment may be performed immediately before, immediately after, and at the same time as the artificial insemination. For example, by mixing the conception chance-improving composition of the present embodiment with semen for artificial insemination, the conception chance-improving composition can be administered simultaneously with artificial insemination.

The ruminant to which the conception chance-improving composition of the present embodiment is administered may be a naturally mated ruminant. In the case of natural breeding, conception chance can also be improved by administering the drug between the estrus day and the one day before and after the estrus day.

The ruminant to which the conception chance-improving composition of the present embodiment is administered may be a ruminant to which a fertilized egg is implanted. The conception chance-improving composition of this embodiment can improve the conception rate of the fertilized egg to be transplanted. During the scheduled pre-transplant estrus date and the period before and after it (e.g., from 5 days before estrus date to 5 days after estrus date), by administering the conception chance-improving composition of this embodiment, it is possible to improve the conception rate of transplanted fertilized eggs.

The conception chance-improving composition of this embodiment can be administered to a ruminant having an endometrial EGF concentration profile abnormality to resolve or reduce the abnormality, thereby improving the conception rate. Thus, the conception chance-improving composition according to the present embodiments can be extensively administered to ruminants in need of improved conception chance, particularly to ruminants that have had an abnormal endometrial EGF concentration profile.

The ruminant to which the conception chance-improving composition of the present embodiment is administered are not specifically limited, and may be any of the ruminants, such as cows, goats, sheep, deer, giraffes, etc. The ruminant to which the conception chance-improving composition of the present embodiment is administered is preferably a cow, goat, or sheep, and particularly preferably a cow. The conception chance-improving composition of the present embodiment is preferably used for dairy cows in which improvement in conception chance is particularly desired.

The conception chance-improving composition of the present embodiment is preferably used, for example, in cows with low conception chance, and is particularly preferably administered to RB cows. Among other things, the composition for conception chance improvement of the present embodiment is preferably administered to a cow whose epidermal growth factor concentration in the endometrium, which is observed in normal conception cows on days 2-4 from estrus, has disappeared or decreased, i.e., a cow with an abnormal endometrial EGF concentration profile, and in particular, more preferably to a cow whose epidermal growth factor concentration in the endometrium on the third day after estrus is less than 4.7 ng/g per tissue weight.

In postpartum ruminants, it may be difficult to conceive due to the recovery state of the uterus. In addition, because the metabolic and physiological conditions related to nutrition differ greatly, they may not respond to treatment with hormone preparations in normal breeding practice. The conception chance-improving composition according to the present embodiment can normalize the EGF concentration profile in the endometrium from after administration to one cycle of estrus, and thus, by administering the conception chance-improving composition of the present embodiment to a ruminant after delivery, improvement in conception rate can be expected. When administered to a postpartum ruminant, it is desirable to administer the conception chance-improving composition in this embodiment before and after the time of the first postpartum insemination. in particular, the number of days after delivery is preferably within the period from the number of days equivalent to two estrous cycles to the number of days equivalent to five estrous cycles; the number of days after delivery is more preferably within the period from the number of days equivalent to two estrus cycles to the number of days equivalent to four cycles; the number of days after delivery is even more preferably within the period from the number of days equivalent to 2.5 estrus cycles to the number of days equivalent to 4 cycles; and the number of days after delivery is further even more preferably within the period from the number of days equivalent to 2.5 estrus cycles to the number of days equivalent to 3.5 cycles. For example, in the case of cows, the conception chance-improving composition of this embodiment is administered preferably within the period of 40 to 100 days after delivery, more preferably within the period of 40 to 90 days, even more preferably within the period of 40 to 80 days, and further even more preferably, the conception chance-improving composition of this embodiment is administered within the period of 45 to 80 days. In particular, it is preferable that the conception chance-improving composition in the present embodiment be administered to a cow within a period of 50 to 80 days after delivery, preferably within a period of 60 to 80 days, and more preferably within a period of 60 to 70 days.

The method for administering the conception chance-improving composition of the present embodiment to the vaginal fornix of the ruminant's vagina or its vicinity can be performed in the same manner as semen administration in artificial insemination. For example, the conception chance-improving composition of this embodiment can be prepared as a solution [that can be] dissolve or dilute as a solution that can be administered to ruminants, and this can be injected into the vagina using a catheter syringe for artificial insemination. Solutions that can be administered to ruminants include solutions that can be administered to the vagina, cervix, and uterus of the ruminants, which include water, buffer, semen of the ruminants, or a solution containing appropriate and pharmaceutically acceptable additives that can be administered to the vagina, cervix, and uterus of the ruminant. The buffer is not particularly limited as long as it can be safely administered to the vagina of ruminants, and examples include PBS (phosphate buffered saline) and physiological saline. As the additive, those similar to those listed above can be used, and additives that are well tolerated in the vagina and endometrium are preferred.

The amount of liquid of the solution of the conception chance-improving composition in this embodiment to be injected into the vaginal portion of the ruminants, used for one treatment is preferably 0.1 to 20 mL. 2 to 20 mL is more preferable, 5 to 20 mL is even more preferable, 10 to 20 mL is even more preferred, and 10 to 15 mL is particularly preferred. If the amount is within the above range, by administering it deep into the vagina, a small amount of peptide can stimulate a wide range of receptors and produce effects. In particular, since the volume of ejaculated semen in a cow's natural state is 2 to 10 mL, in the case of a cow, it is particularly preferable to administer the ejaculate in a volume within the above range.

In addition, the amount of active ingredient of the conception chance-improving composition of the present embodiment in the solution may be sufficient to normalize the endometrial EGF concentration profile and improve the conception rate, for example, the concentration of the peptide or polypeptide of the active ingredient may be 40 nmol or more per treatment solution, preferably 50 nmol or more, more preferably 100 nmol or more, even more preferably 200 nmol or more, and further even more preferably 320 nmol or more. The upper limit of the concentration of the peptide or polypeptide of the active ingredient per treatment solution is not specifically limited, and can be, for example, 2400 nmol or less, preferably 2000 nmol or less, and more preferably 1600 nmol or less.

By using an artificial insemination straw filled with the conception chance-improving composition of the present embodiment, the conception chance-improving composition can be more easily administered into the vagina of a ruminant. Preferably, the conception chance-improving composition of the present embodiment to be filled into an artificial insemination straw is a solution in which the peptide or polypeptide of the active ingredient is dissolved in 0.1 to 20 mL of a solution that can be administered to ruminants. The solution that can be administered to ruminants can be the same as the one listed above, and the amount of the solution that dissolves the active ingredient is preferably 2 to 20 mL, more preferably 5 to 20 mL, even more preferably 10 to 20 mL, and further even more preferably 10 to 15 mL. The use of artificial insemination seminal fluid as the solution or portion thereof results in an artificial insemination straw capable of performing both administration of the conception chance-improving composition and artificial insemination in this embodiment.

### Examples

While the present invention is described in further detail in the following examples, the present invention is not limited to the following examples.

### <Test cow>

All of the cows subjected to the subsequent experiments were Holstein-type, postnatally lactated cows reared on commercial farms in the Hokkaido area of Japan (order of birth: 2-6 cows, age: 3-9 years, milk yield: >9500 kg/305 days of fatcorrected milk). Cows that had problems requiring at least three veterinarian consultations at the time of delivery and during delivery were excluded from the study.

### <Measurement of Endometrial EGF Concentration in Cows>

Measurement of the endometrial EGF concentration was performed according to the method described in the Non-patent Literature 1. Specifically, it was conducted as follows.

### (1) Endometrial sample collection

After washing the external genitalia of a cow that had been given caudal epidural anesthesia, a biopsy instrument (manufactured by Fujihira Industry) was inserted into the vagina to collect samples. The harvested endometrium tissue was weighed and then frozen in liquid nitrogen within 10 minutes after collection and stored at -80°C until an EGF assay was performed.

### (2) Preparation of measurement samples (endometrial tissue extracts) for the EGF assay

First, the tissue samples were shredded, 1 M acetic acid (5 mL/g of tissue), homogenized in ice cooled, and the homogenate was centrifuged. The supernatant after centrifugation was collected and stored, and the remaining pellets were homogenized again and centrifuged to collect the supernatant. The first and second supernatants were combined and concentrated. The resulting concentrated samples were applied to a Sephadex G-50 column (manufactured by Amasham Biosciences) and eluted with 10 mM PBS (pH 7.2) to perform the size fractionation. Fractions containing more than 10k protein were combined into one sample, freeze-dried, and stored at -80 °C. Frozen samples were dissolved and reconstituted in PBS on the day of the EGF assay.

### (3) Measurement of EGF assay

The EGF concentration of the endometrial tissue extract was measured by ELISA using a 96-well plate. All assays were performed on three independent trials. Anti-human EGF mouse monoclonal antibody (clone: MAB636, manufactured by R & D Systems) was used as the solid phase antibody and anti-human EGF rabbit antiserum (Product No.: 5022-100, manufactured by Biogenesis Ltd) was used as the detection antibody. The assay sensitivity was 10 pg/well. The coefficient of variation within and between assays at 50 pg/well was 4.2% and 6.8%, respectively.

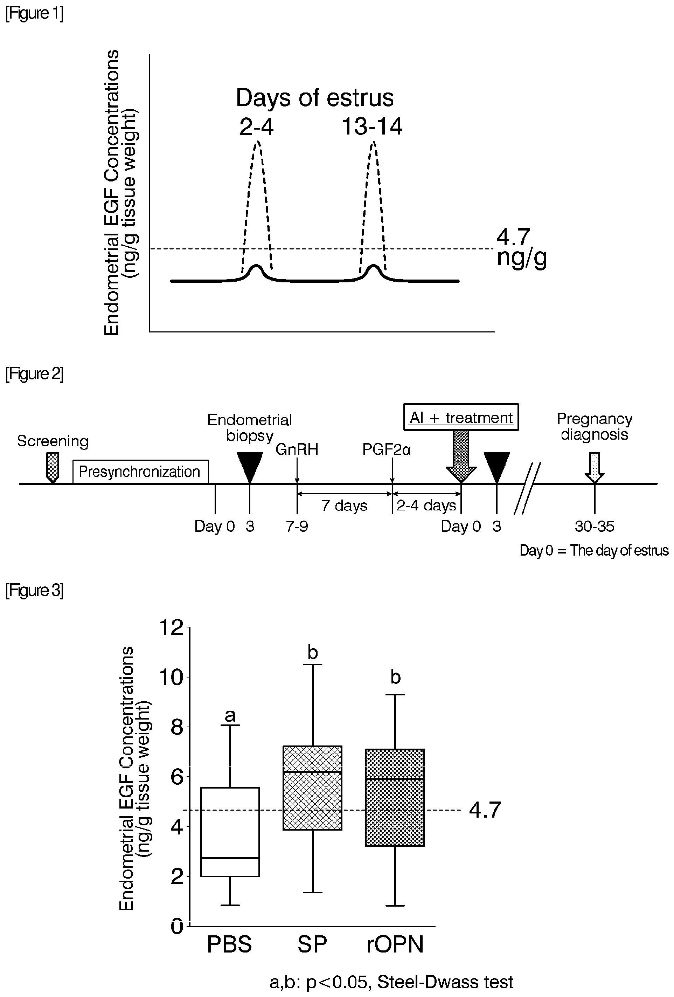

FIG.1 schematically shows changes (solid lines) over time in the endometrial EGF concentration of RB cows after estrus. In normal cows, peaks with an increase in EGF concentration are observed on days 2-4 and 13-14 of estrus (dotted line in the drawing). In contrast, the majority of RB cows show an endometrial EGF concentration of 4.7 ng/g of tissue or less on days 2-4 and 13-14 of estrus, indicating disappearing or decreasing peak concentration.

In the subsequent studies, the endometrial EGF concentration was measured on the third day (the day on which estrus or artificial insemination (AI) was performed was considered Day 0). The endometrial EGF concentration profile was considered normal if the endometrial EGF concentration was within the normal range on the third day (4.70 to 13.50 ng/g of tissue).

### <Synchronization of ovulation>

Ovulation in RB cows was synchronized by Ovsynch protocol, Ovsynch protocol with E₂ (estradiol) treatment, or Ovsynch protocol with P₄ (progesterone) supplementation. In the Ovsynch protocol, the bovine was first treated with gonadotropin-releasing hormone (GnRH) (100 *µg* of fertirelin acetate intramuscularly, Conseraru [sic. transliteration] (registered trademark) Injection, product of MSD Animal Health), continued with PGF_{2*α*} (25 mg of dinoprostrometamine muscle injection) 7 days later, and approximately 55 ± 2 hours after PGF_{2*α*} treatment, a second dose of GnRH (100 mg fertirelin acetate intramuscularly) was administered. In the Ovsynch protocol with E2 treatment, in addition to the Ovsynch protocol procedure, 1 day after PGF_{2*α*} treatment, [the cow] was treated with estradiol benzoate (1 mg intramuscular estradiol injection, Kyoritsu Seiyaku Corporation). In the Ovsynch protocol with P₄ supplementation, on the day of the first GnRH treatment, in addition to the Ovsynch procedure, an intravaginal indwelling P₄ formulation vaginal (CIDR) containing 1.9 g of P₄ (Product No.: CIDR 1900, manufactured by Zoetis Japan) was inserted. CIDR was removed 7 days after insertion. The Ovsynch protocol and the Ovsynch protocol with E₂ were started between Days 5 and 10 or Days 18 and 20 of the estrus cycle. The Ovsynch protocol with P₄ supplementation was initiated on days 13 and 17. When cows were synchronized to ovulate, the next day of the second GnRH treatment was considered day 0.

### <Artificial insemination (AI)>

Artificial insemination was performed by a professional AI technician using commercially available frozen and thawed Holstein semen.

### <Injection of treatment solution into the vagina>

All treatment solutions (samples) were prepared to a volume of 10 mL using PBS. The samples were filled into a 10 mL syringe and injected into the vagina. A disposable plastic catheter for AI was attached to the syringe filled with the sample to be injected, and the tip of the plastic catheter was introduced into the vagina. The tip of the catheter was guided deep into the vagina adjacent to the external opening of the cervix using an AI method. The syringe plunger was then pressed to inject the sample. The sample was injected into the vagina immediately after the AI.

### [Reference Example 1]

OPNs have many post-translational modification sites and are well known as proteins that express various functions through these modifications. Therefore, we first confirmed the effect of bovine full-length recombinant OPN (rOPN) produced by Escherichia coli, which does not have post-translational modifications, on normalizing the EGF concentration profile and conception chance of RB cattle.

### <Test cow>

Among the RB cows in the lactation period of the Holstein species, the RB cows that were confirmed to have an abnormal endometrial EGF concentration profile, i.e., confirmed to have an endometrial EGF concentration of less than 4.7 ng/g of tissue on estrus day 3 were treated as test cows (n = 216, number of days after delivery: 120-180 days).

RB cows were selected by local veterinarians using criteria of no detectable abnormalities in the estrous cycle length, clinical signs, genital, and failure to become pregnant after at least three artificial inseminations. Prior to the study, all RB cows were confirmed to have no abnormalities in their uterine condition by trans-rectal ultrasound and cytobrush technique.

### <Preparation of rOPN>

rOPNs were prepared by E. coli expression system to generate OPNs without post-translational modifications. Specifically, a transformant was obtained by introducing into E. coli a gene consisting of a base sequence that encodes a putative mature protein of bovine OPN (protein consisting of the region 17 to 278 of the amino acid sequence of SEQ ID NO: 4).

The genes encoding bovine OPN (Uniprot ID: P31096) were synthesized by "GeneArt^{®} gene synthesis" (provided by Thermo Fischer Scientific) using codon optimization for E. coli expression. DNA encoding the putative mature protein region (region from 17th leucine to 278th asparagine in the amino acid sequence of SEQ ID NO: 4) of OPN was amplified by PCR using the primers listed in Table 1 and DNA polymerase "KOD Plus Neo (registered trademark), manufactured by Toyobo Co., Ltd.". In the base sequence of Table 1, the underlined portion in the forward primer (sense strand) indicates the Ndel site, and the underlined portion in the reverse primer (antisense strand) indicates the Hind III site.

**[Table 1]**

| Primer | Base sequence | SEQ ID NO: |
|---|---|---|
| Forward (sense) | 5' -GCCGGCGGCAGCATATG CTGCCGGTTAAACCGACCAG-3' | 5 |
| Reverse (antisense) | 5' -CGAGTGGGCCAAGCTTTTAATTCACTTCACTGCTTG-3' | 6 |

The PCR product was purified using a "QIAEX II Gel Extraction Kit" (manufactured by Qiagen) and then inserted into the Ndel-Hind III site of plasmid pET - 28a (manufactured by Merck Millipore) using SLiCE (Seamless Ligation Cloning Extract). The resulting plasmid vector was named SPP1/pET-28a. The vector produces a recombinant protein comprising an amino acid sequence from a plasmid vector comprising a histidine tag (MGSSHHHHHSSGLVPRGSHM: SEQ ID NO: 7) on the N-terminal side of the predicted mature protein region of the OPN (17-278 region of SEQ ID NO:4).

E. coli Rosetta (DE3) carrying SPP1/pET - 28a (manufactured by Merck Millipore) was incubated at 37°C on an LB agar plate containing 50 *µ*g/mL kanamycin and 30 *µg*/mL chloramphenicol. The resulting single colony was inoculated into 150 mL of LB medium containing 50 *µ*g/mL kanamycin and 30 *µ*g/mL chloramphenicol, and grown with shaking at 37°C for one night, and used as a seed culture. Seed cultures were inoculated into 3000 mL of LB medium containing 50 *µ*g/mL kanamycin and grown at 37°C. When the OD₆₀₀ of the medium reached 0.4-0.8, the medium was cooled in ice for 10 minutes and protein expression was induced by adding 0.1 mM IPTG (*isopropyl β-D-* thiogalactpyranoside). After that, it was cultured at 30°C for 20-22 hours. The resulting culture was centrifuged (10,000 × g, 25°C, 5 minutes) to collect E. coli and frozen at -80°C until rOPN was purified.

rOPN was purified by the following method. E. coli was resuspended in buffer A (20 mM sodium phosphate buffer, pH 7.0, 0.3 M NaCl) at 4°C and ground by ultrasound before centrifugation (4°C, 6,000 x g, 20 min) to obtain a cell-free lysate. The lysate was applied to "Ni²⁺ -charged Chelating Sepharose Fast Flow column" (product of Cytiva). The column was washed with buffer A containing 20 mM imidazole, and rOPN was then eluted with a gradient solution prepared so as to have a linear gradient of 20 to 500 mM imidazole in buffer A. The purified rOPN was dialyzed on PBS (product of Takara Bio) with a cellulose membrane (cellulose tube (30/32), product of Viskase Companies). rOPN purification and dialysis were performed at 4°C. After addition of 1% (w/v) trehalose as a cryoprotectant to dialyzed protein solution containing rOPN, the solution was filtered (filter pore size: 0.22 *µ*m), and the solution containing 0.3 mg of rOPN was dispensed into 10 mL vials and freeze-dried. Freeze-dried rOPNs were stored at 4°C until injection into the cow. It should be noted that the concentration of rOPN in the dialysis protein solution was calculated by calculating the crude protein concentration by ultraviolet absorption photometry, and then correcting the concentration by the purity evaluated by polyacrylamide gel electrophoresis. Also, 0.3 mg rOPN contains 16 nmol rOPN when quantified based on amino acid analysis.

### <Treatment with OPN>

For OPN treatment, 0.3 mg of rOPN was dissolved in 10 mL of PBS (rOPN treatment solution) and the solution was injected into the vagina. As a positive control, 0.5 mL of seminal plasma was diluted with PBS to prepare 10 mL of the solution (SP treatment solution). PBS was used as a negative control.

The schedule for OPN treatment is shown in FIG. 2. First, all cows were screened to confirm they were RB cows and then their estrus was synchronized. An endometrium biopsy was then performed on the third day after estrus. The cows that were confirmed to have an abnormal endometrial EGF concentration profile based on this endometrial biopsy were treated as the test cows. Next, on days 7 to 9 after estrus, GnRH was administered, and 7 days later, prostaglandin F2*α* (PGF2*α*) was administered to synchronize estrus. Estrus was confirmed 2 to 4 days after administration of PGF2. OPN treatment was performed with artificial insemination (AI) within 4 to 12 hours of estrus confirmation. PBS treatment was performed on 74 cows, SP treatment was performed on 37 cows, and rOPN treatment was performed on 105 cows.

The date of artificial insemination and OPN treatment was defined as the day of estrus, and on the third day thereafter, the endometrial EGF concentration was measured again. Furthermore, on the 30th to 35th day from the day of estrus, a pregnancy test was performed, and the conception rate was calculated and compared using Fisher's exact test. Pregnancy was diagnosed by transrectal ultrasound examination.

**[Table 2]**

| Groups | Normalization rate (%) | EGF profile after treatment | Conception rate (%) | |
|---|---|---|---|---|
| PBS | 29.7 ^{a} (22/74) | Normalized | 47.4 | (9/19) |
| | | Unnormalized | 18.4 | (7/39) |
| | | total | 28.1^{a} | (16/58) |
| SP | 62.3 ^{b} (23/37) | Normalized | 66.7^{A} | (6/9) |
| | | Unnormalized | 27.3^{B} | (3/11) |
| | | total | 45.0^{ab} | (9/20) |
| rOPN | 58.1 ^{b} (61/105) | Normalized | 68.1^{A} | (32/47) |
| | | Unnormalized | 19.4^{B} | (7/36) |
| | | total | 47.0^{b} | (39/83) |

| | | | | |
|---|---|---|---|---|
| a,b : p<0.05, Fisher's exact test with BH method, A,B: p<0.05, Fisher's exact test, | | | | |

The measurement results of EGF concentration and the conception rate (%) on the third day after treatment of the groups subjected to each treatment are shown in FIG. 3 and Table 2. As shown in FIG.3 and Table 1, rOPN treatment improved the EGF concentration on the third day after estrus to a degree similar to SP treatment, confirming that rOPN normalized the abnormal endometrial EGF concentration profile. Furthermore, in both groups, the conception rate in cows with normalized EGF concentration profiles was higher than in cows without normalization. As a result, it was confirmed that the rOPN treatment group showed significantly higher conception rates compared to the PBS treatment group, and that the rOPN treatment improving conception chance.

### [Example 1]

OPNs are proteins with multiple physiologically active sites that modulate a variety of life events. Therefore, an attempt was made to identify the partial regions of OPN that were involved in resolving the abnormal endometrial EGF concentration profile.

### <Test cow>

Similar to reference Example 1, a Horstein RB cow with an abnormal endometrial EGF concentration profile was designated as a test cow (n=294).

### <Preparation of partial peptides and partial proteins of OPN>

The OPN has a thrombin cleavage site (the 162nd serine of the amino acid sequence of SEQ ID NO: 4), which results in the generation of an N-terminal fragment (the 17th-161st amino acid region of the amino acid sequence of SEQ ID NO: 4) and a C-terminal fragment (the 162nd -278th amino acid region of the amino acid sequence of SEQ ID NO: 4). The N-terminal fragment has an integrin binding domain. The integrin binding domain consists of two integrin binding motifs: an N-terminal RGD motif (the 152-154th region of the amino acid sequence of SEQ ID NO: 4) and a C-terminal SVAYGLK motif (the 155-161th region of the amino acid sequence of SEQ ID NO: 1 : SEQ ID NO: 4). These two integrin binding motifs differ in the type of integrin they bind to each other. The C-terminal fragment also targets CD44, but the binding domain is unknown.

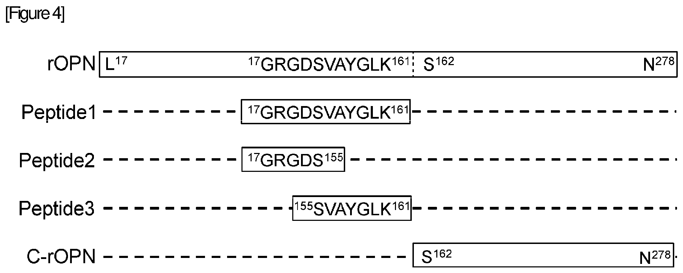

Peptides and polypeptides composed of amino acid sequences shown in FIG. 4 were synthesized based on the domain structure of OPN. Peptide 1 (SEQ ID NO: 2: GRGDSVAYGLK) is a peptide of the 151-161 region of the amino acid sequence of SEQ ID NO: 4; Peptide 2 (SEQ ID NO. 3: GRGDS) is a peptide of the 151-155 region of the amino acid sequence of SEQ ID NO: 4; and Peptide 3 (SEQ ID NO. 1: SVAYGLK) is a peptide of the 155-161 region of the amino acid sequence of SEQ ID NO: 4).

Peptide 1 and peptide 3 were synthesized in the form of trifluoroacetate (manufactured by Peptide Institute, Inc.). Peptide 2 was left as is in the form of fibronectin active fragment (GRGDS) in acetate (manufactured by Peptide Institute, Inc.). Each peptide provided as the freeze-dried peptide was dissolved in sterile ultrapure water and filtered (filter pore size: 0.22 *µ*m). The solution containing the filtered peptide was taken as a peptide sample and dispensed into vials of 32 nmol, 320 nmol, and 1600 nmol each and freeze-dried. The freeze-dried peptide were stored at 4°C until injection into the cow. The amount of peptide was quantified based on amino acid analysis.

rOPN used in reference Example 1 was used. C - rOPN (the 162-278th region of the amino acid sequence of SEQ ID NO: 4) used a recombinant protein synthesized with E. coli, similar to rOPN. Each purified recombinant protein was freeze-dried and stored at 4°C until use. Protein amounts were quantified based on amino acid analysis.

### <Treatment with OPN>

Of the OPN treatment, rOPN treatment was used as a positive control with 0.3 mg of rOPN dissolved in 10 mL of PBS as the treatment solution. C - rOPN treatment was a treatment solution in which 28 nmol or 46 nmol C - rOPN was dissolved in 10 mL of PBS. In peptide 1 treatment, a solution in which 32 nmol, 320 nmol, or 1600 nmol of peptide 1 was dissolved in 10 mL of PBS was used as the treatment solution. In peptide 2 treatment, a solution in which 320 nmol, or 1600 nmol of peptide 2 was dissolved in 10 mL of PBS was used as the treatment solution. In peptide 3 treatment, a solution in which 320 nmol, or 1600 nmol of peptide 3 was dissolved in 10 mL of PBS was used as the treatment solution. PBS was used as a negative control.

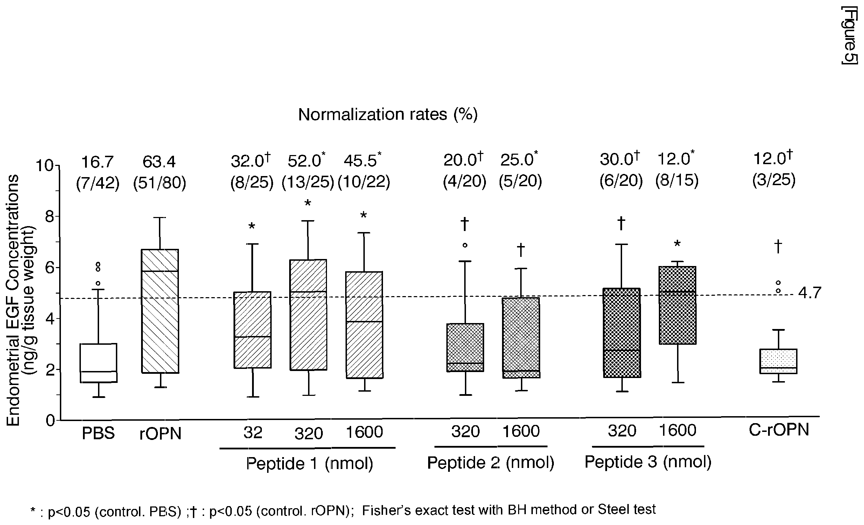

OPN treatment was scheduled in the same manner as Reference Example 1 (FIG. 2), except that estrus synchronization for artificial insemination and OPN treatment was changed to ovulation synchronization and artificial insemination was performed at 16-20 hours after the second GnRH treatment in ovulation synchronization treatment. The results of the measurement of EGF concentration on the third day after treatment in the groups treated with each treatment are shown in FIG. 5, and the results of conception rate (%) are shown in Table 3. In FIG. 5, the number at the top of each column represents the normalization rate of the EGF concentration profile ([number of cows with EGF concentration of 4.70 ng /g of tissue or higher]/[number of cows treated] x 100%).

**[Table 3]**

| Endometrial EGF profile after treatment | PBS | rOPN | Peptide1 (nmol) | | | Peptide2 (nmol) | | Peptide3 (nmol) | | C-rOPN (nmol) | Total |
|---|---|---|---|---|---|---|---|---|---|---|---|
| | | | 32 | 320 | 1600 | 320 | 1600 | 320 | 1600 | 28 or 46 | |
| Normalized | 57.1 (4/7) | 62.0^{a} (31/50) | 37.5 (3/8) | 69.2^{a} (9/13) | 60.0 (6/10) | 75.0^{a} (3/4) | 60.0 (3/5) | 83.3^{a} (5/6) | 62.5^{a} (5/8) | 33.3 (1/3) | 61.4^{a} (70/114) |
| Unnormalized | 8.8 (3/34) | 21.4^{b} (6/28) | 11.8 (2/17) | 16.7^{b} (2/12) | 45.5 (5/12) | 6.7^{b} (1/15) | 20.0 (3/15) | 14.3^{b} (2/14) | 28.6^{b} (2/7) | 13.6 (3/22) | 16.5^{b} (29/176) |
| Total | 17.1 (7/41) | 47.4 (37/78) | 20.0^{†} (5/25) | 44.0* (11/25) | 50.0* (11/22) | 21.1^{†} (4/19) | 30.0 (6/20) | 35.0 (7/20) | 46.7* (7/15) | 16.0^{†} (4/25) | 41.0 (119/290) |

| | | | | | | | | | | | |
|---|---|---|---|---|---|---|---|---|---|---|---|
| * : p<0.05 (vs. PBS); † : p<0.05 (vs. rOPN); Fisher's exact test in same row a,b : p<0.05; Fisher's exact test in the same column | | | | | | | | | | | |

As shown in FIG. 5, improvement in the endometrial EGF concentration on the third day after estrus was confirmed in the 32 to 1600 nmol treated group of peptide 1 and in the 1600 mol treated group of peptide 3, the same extent as in the rOPN treated group. In addition, improvement in the EGF concentration profile on the third day after estrus was confirmed to be the same as in the rOPN treatment group in the 320 to 1600 nmol treatment group for peptide 1 and the 1600 mol treatment group for peptide 3. On the other hand, for the other samples, the normalization rates of the endometrial EGF concentration and EGF concentration profiles were lower than in the rOPN group, which was comparable to the PBS group. Also, as shown in Table 2, in all groups, the cows with normal EGF concentration profiles exhibited a higher conception rate than the cows with abnormal profiles. In addition, the conception rate in each group was higher in the 320 to 1600 nmol treated group for peptide 1 and the 1600 nmol treated group for peptide 3 than in the control PBS treated group, as in the rOPN treated group. On the other hand, the overall conception rate in the 1600 nmol treated group for peptide 2 and the 320 nmol treated group for peptide 3 was about halfway between the PBS treated group and the rOPN treated group. The 320 nmol peptide 2 treated group and the C-rOPN treated group had similar conception rates to the PBS treated group. These results showed that the integrin-binding domain is involved in the normalization of the endometrial EGF concentration profile and the improvement of conception chance by OPN, and in particular, the SVAYGLK motif plays an important role.

### [Example 2]

We investigated the effect of the integrin-binding domain of OPN on normalizing the endometrial EGF concentration profile and restoring conception chance in RB cows.

### <Test cow>

Similar to reference Example 1, a Horstein RB cow with an abnormal endometrial EGF concentration profile was designated as a test cow (n=586).

### <Preparation of partial peptides and partial proteins of OPN>

rOPN and peptides 1-3 prepared in Example 1 were used.

As peptide 4, a peptide (SEQ ID NO: 8) in which the fourth D (aspartic acid) of peptide 1 was replaced with E (glutamic acid) was used. This amino acid substitution mutation changes the RGD motif to an RGE sequence that does not have integrin binding activity. Peptide 4 was synthesized in the form of trifluoroacetate (manufactured by Peptide Institute, Inc.), and a peptide sample was prepared in the same manner as peptide 1.

### <Treatment with OPN>

Treatment with OPN was performed in the same manner as Example 1. The following treatments were each administered at the time of artificial insemination: rOPN (0.3 mg, n = 111), peptide 1 (GRGDSVAYGLK) (32 nmol, 320 nmol, or 1600 nmol n = 25, 128, and 50, respectively), peptide 2 (GRGDS) (320 nmol or 1600 nmol each, n = 20), peptide 3 (SVAYGLK) (320 nmol or 1600 nmol, n = 25 and 55, respectively), peptide 4 (GRGESVAYGLK) (320 nmol or 1600 nmol, n = 50 and 26, respectively) and PBS (n = 77).

**[Table 4]**

| Groups | Dose | (n) | Normalization rate, % (n/n) | Endometrial EGF Concentrations (ng/g tissue weight) | |
|---|---|---|---|---|---|
| | | | | Before treatment | After treatment |
| PBS | - | (77) | 20.8 (16/77) | 1.54 (1.20-1.96) ^{a} | 2.16 (1.57-3.61) ^{b} |
| rOPN | 0.3 mg | (111) | 61.3 (68/111) | 1.64 (1.37-1.93) ^{a} | 5.33 (2.18-6.23) ^{b} |
| Peptide 1 | 32 nmol | (25) | 32.0 (8/25) ^{†} | 1.68 (1.41-2.35) ^{a} | 3.26 (2.04-5.01) ^{b} |
| | 320 nmol | (128) | 49.2 (63/128) * | 1.71 (1.36-2.33) ^{a} | 4.38 (1.85-5.97) ^{b*} |
| | 1600 nmol | (50) | 52.0 (26/50) * | 1.67 (1.29-1.92) ^{a} | 4.90 (2.56-6.22) ^{b*} |
| Peptide 2 | 320 nmol | (20) | 20.0 (4/20) ^{†} | 1.89 (1.56-2.27) ^{a} | 2.17 (1.84-3.74) ^{b†} |
| | 1600 nmol | (20) | 20.0 (4/20) ^{†} | 1.67 (1.43-1.81)^{a} | 1.83 (1.61-4.73) ^{b†} |
| Peptide 3 | 320 nmol | (25) | 36.0 (9/25) ^{†} | 1.43 (1.24-1.95) ^{a} | 2.67 (1.45-5.44) ^{b†} |
| | 1600 nmol | (55) | 50.9 (28/55) ^{*} | 1.64 (1.39-1.96) ^{a} | 4.92 (2.36-6.03) ^{b*} |
| Peptide 4 | 320 nmol | (50) | 16.0 (8/50) ^{†} | 1.50 (1.17-1.83) ^{a} | 2.44 (1.83-3.56) ^{b†} |
| | 1600 nmol | (26) | 19.2 (5/26) ^{†} | 2.25 (1.66-2.92) ^{a*†} | 2.83 (2.18-4.12) ^{b†} |

| | | | | | |
|---|---|---|---|---|---|
| *: p<0.05 (control. PBS, in same row,) † : p<0.05 (vs. rOPN, in same row), a,b : p<0.05 (in the same column) | | | | | |

Table 4 shows the measurement results of the normalization rate of the endometrial EGF concetration and EGF concentration profile in each treatment group. As shown in Table 4, normalization rates were higher in the groups treated with peptide 1 or 3 than in the PBS treatment group. In particular, in the 320 to 1600 nmol treated group for peptide 1 and the 1600 nmol treated group for peptide 3, as in the rOPN treated group, the normalization rate of the endometrial EGF concentration and the EGF concentration profile were significantly higher than in the control PBS treated group. On the other hand, in the peptide 2 treatment group, no improvement in endometrial EGF concentration or normalization of EGF concentration profile was observed. These results confirmed the followings: The administration of peptide 1 or peptide 3 normalizes the endometrial EGF concentration profile, similar to administration of rOPN; this effect is demonstrated by peptide 1 at a lower concentration than peptide 3; and peptide 3 (i.e., the SVAYGLK motif) is the minimum functional unit of OPN's ability to normalize the endometrial EGF concentration profile.

On the other hand, in the treatment group of peptide 4, in which amino acid substitution mutations were introduced into peptide 1 to inactivate the integrin binding activity of the RGD motif, no improvement of the endometrial EGF concentration and normalization of the EGF concentration profile observed in peptide 3 were confirmed. These results suggest that the RGD motif is also involved in the normalization of endometrial EGF concentration by the SVAYGLK motif.

**[Table 5]**

| Groups | Dose | Endometrial EGF profile after treatment | (n) | Endometrial EGF Concentrations (ng/g tissue weight) | | Conception Rate (%) |
|---|---|---|---|---|---|---|
| | | | | Before treatment | After treatment | |
| PBS | | Normalized | (16) | 1.42 (1.17-1.90)^{a} | 5.31 (5.06-6.02) ^{bA} | 56.3 (9/16) ^{C} |
| | | Unnormalized | (61) | 1.58 (1.29-1.96)^{a} | 1.85 (1.47-2.64) ^{b} | 11.9 (7/58) ^{D} |
| | | Total | (77) | 1.54 (1.24-1.94)^{a} | 2.16 (1.57-3.61)^{bA} | 21.6 (16/74)^{A} |
| rOPN | 0.3 mg | Normalized | (68) | 1.64 (1.37-2.10)^{a} | 6.07 (5.41-6.168) ^{bB} | 64.2 (43/67) ^{C} |
| | | Unnormalized | (43) | 1.64 (1.43-1.84)^{a} | 1.83 (1.58-2.76) ^{b} | 20.9 (9/42) ^{D} |
| | | Total | (111) | 1.64 (1.37-1.93) ^{a} | 5.33 (2.18-6.23) ^{bB} | 47.7 (52/109) ^{B} |
| Peptide 1 | 320 nmol | Normalized | (63) | 1.82 (1.39-2.51) ^{a} | 5.97 (5.38-6.71) bAB | 60.3 (38/63) ^{C} |
| | | Unnormalized | (65) | 1.63 (1.30-2.06) | 1.86 (1.58-3.18) | 23.1 (15/65) ^{D} |
| | | Total | (128) | 1.71 (1.36-2.33) ^{a} | 4.38 (1.85-5.97) ^{bB} | 41.4 (53/128) ^{B} |
| | 1600 nmol | Normalized | (26) | 1.78 (1.37-1.94) ^{a} | 6.18 (5.55-7.39) ^{bB} | 65.4 (17/26) ^{C} |
| | | Unnormalized | (24) | 1.43 (1.24-1.85) ^{a} | 2.50 (1.43-3.45) ^{b} | 33.3 (8/24) ^{D} |
| | | Total | (50) | 1.67 (1.29-1.92) ^{a} | 4.90 (2.56-6.22) ^{bB} | 50.0 (25/50) ^{B} |
| Peptide 3 | 1600 nmol | Normalized | (28) | 1.55 (1.28-1.91)^{a} | 6.00 (5.26-6.21) ^{bAB} | 64.3 (18/28) ^{C} |
| | | Unnormalized | (27) | 1.67 (1.30-2.46) ^{a} | 2.36 (1.47-3.39) ^{b} | 18.5 (5/27) ^{D} |
| | | Total | (55) | 1.64 (1.39-1.96) ^{a} | 4.92 (2.36-6.03) ^{bB} | 41.8 (23/55) ^{B} |

| | | | | | | |
|---|---|---|---|---|---|---|
| A, B: p < 0.05 (in cows with the same EGF profile in same column: EGF concentrations and conception rate) C, D: p < 0.05 (in cows within the same group in same column), a, b: p < 0.05 (in same raw) | | | | | | |

Table 5 show the results of a conception chance study of the treatment groups in which an improvement in the normalization rate of the endometrial EGF concentration profile was confirmed. As a result, conception chance improved in all peptide and treatment groups to the same extent as in the rOPN treatment group.

### [Example 3]

We investigated the effects of rOPN on the endometrial EGF concentration profile and conception chance in cows during the first postpartum insemination.

### <Test cow>

The following cows were used as test cows (n = 106): A Holstein cow at the time of first insemination after calving, and cows with no detectable abnormalities in estrus cycle, clinical signs, uterine cytology, vaginal examination, rectal examination, and genital examination by ultrasound prior to study inclusion.

### <Treatment with OPN>

OPN treatment was performed at the initial insemination 60-70 days after delivery. Of the test cows, only artificial insemination was performed in 52 cows (untreated group), and rOPN (16 nmol) was given in 54 cows (rOPN treatment group). Endometrial EGF concentration was measured on the third day from the artificial insemination date. Pregnancy test was performed by transrectal ultrasound examination between 30 and 35 days after the day of artificial insemination.

**[Table 6]**

| Groups | (n) | The proportion of cows with the normal EGF profile (%) | Endometrial EGF Concentrations (ng/g tissue weight) | Conception rate (%) |
|---|---|---|---|---|
| No treatment | (52) | 65.4 (34/52) ^{A} | 5.15 (3.08-5.81)^{A} | 44.2 (23/52) ^{A} |
| rOPN | (54) | 81.5 (44/54) ^{B} | 5.98 (5.15-6.81) ^{B} | 63.0 (34/54) ^{B} |

| | | | | |
|---|---|---|---|---|
| A, B: p < 0.05 (between groups) | | | | |

Table 6 shows the measurement results of the endometrial EGF concentration, normalization rate, and conception rate in each treatment group. The results showed that in the rOPN-treated group, the endometrial EGF concentration was higher than in the untreated group, and the normalization rate was improved, and the conception rate was also improved.

### [Example 4]

We investigated the effects of rOPN and peptide 1 on the endometrial EGF concentration profile and conception chance in cows during the first postpartum insemination.

### <Test cow>

Similar to Example 3, the following cows were used as test cows (n = 220): Holstein cows that are the first to be artificially inseminated after delivery, and cows with no detectable abnormalities in estrus cycle, clinical signs, uterine cytology, vaginal examination, rectal examination, and genital examination by ultrasound prior to study inclusion.

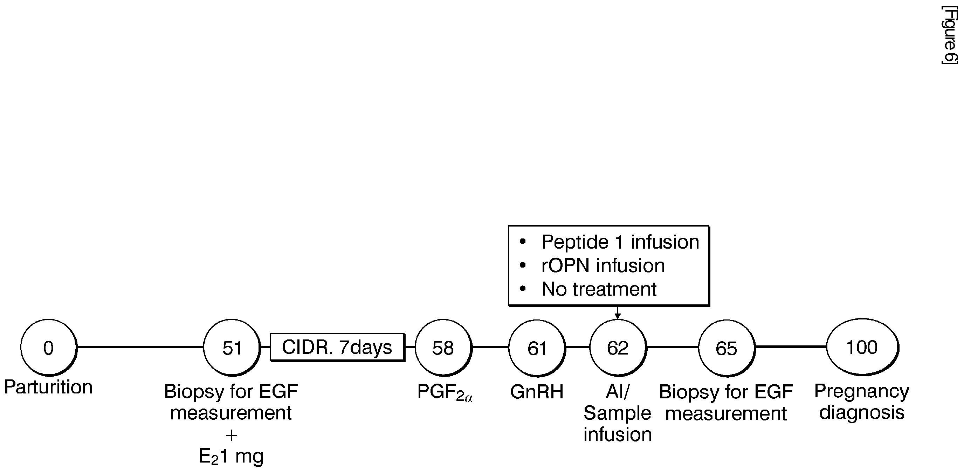

A schedule for OPN treatment is shown in FIG. 6. A biopsy to measure endometrial EGF concentration on the third day of the estrus was performed on postpartum day 51. Next, we synchronized ovulation in cows. Cows are artificially inseminated on the 62nd day after delivery, at the same time, rOPN (16 nmol, n = 41) or peptide 1 (GRGDSVAYGLK, 320 mol, n = 97) was injected vaginally. In the untreated group (n = 82), only artificial insemination was performed. The second measurement of the endometrial EGF concentration was performed on the third day (Day 65 after delivery) from the date of artificial insemination. Pregnancy was assessed by transrectal ultrasound around 38 days (around 100 days after delivery) from the artificial insemination date.

**[Table 7]**

| Groups | The indicated EGF profile before treatment (n) | The proportion of cows with the normal EGF profile after treatment (%) | Endometrial EGF Concentrations (ng/g tissue weight) | | | The proportion of cows whose EGF concentrations increased(%) | Conception Rate (%) |
|---|---|---|---|---|---|---|---|
| | | | Before treatment | After treatment | ΔEGF | | |
| No treatment | Normal (43) | 93.0 (40/43) ^{A} | 5.46 (5.03-6.21) ^{aA} | 5.84 (5.27-6.38) ^{a} | 0.14 (-0.29-0.83) ^{a} | 58.1 (25/43) ^{a} | 37.2 (16/43) |
| | Altered (39) | 23.1 (9/39) ^{A} | 1.76 (1.44-2.50) ^{bAx} | 3.15 (2.46-4.26) ^{bAy} | 1.40 (0.48-2.67) ^{bA} | 89.7 (35/39) ^{b} | 18.0 (7/39) ^{A} |
| | Total | 59.8 (49/82) | 4.78 (1.78-5.61) ^{a} | 5.04 (3.17-6.02) ^{b} | 0.57 (-0.04-1.42) | 73.2 (60/82) | 28.0 (23/82) ^{A} |
| rOPN | Normal (18) | 66.7 (12/18) ^{B} | 5.01 (4.87-5.22) ^{aB} | 5.26 (4.81-5.62) | 0.21 (-1.59-1.12)^{a} | 55.6 (10/18)^{a} | 44.4 (8/18) |
| | Altered (23) | 78.3 (18/23) B | 3.69 (2.38-4.26) ^{bBx} | 5.22 (3.84-6.06) ^{By} | 1.52 (0.90-2.86) ^{bA} | 91.3 (21/23) ^{b} | 47.8 (11/23) ^{B} |
| | Total | 73.2 (30/41) | 4.56 (3.62-5.00) ^{a} | 5.22 (4.57-5.91)^{b} | 1.06 (-0.01-2.45) | 75.6 (31/41) | 46.3 (19/41)^{AB} |
| Peptide 1 | Normal (49) | 71.4 (35/49) B | 5.66 (5.06-6.26) ^{aAx} | 5.62 (4.32-6.06) ^{ay} | -0.29 (-0.99-0.62) ^{a} | 34.7 (17/49) ^{a} | 32.7(16/49) ^{a} |
| | Altered (48) | 54.2 (26/48) ^{B} | 2.58 (1.84-2.97) bAx | 4.92 (3.98-5.74) bBy | 2.21 (1.20-3.32) ^{bB} | 93.4 (45/48) ^{b} | 60.4 (29/48) ^{bB} |
| | Total | 62.9 (131/97) | 4.70 (2.58-5.67) ^{a} | 5.16 (4.14-5.92) ^{b} | 0.80 (-0.36-2.18) | 63.9 (62/97) | 46.7 (45/97) ^{A} |

| | | | | | | | |
|---|---|---|---|---|---|---|---|
| a, b: p < 0.05 (between the different EGF profile before treatment in the same group), A,B: p < 0.05 (between groups in the same EGF profile before treatment), x, y: p < 0.05 (between before and after treatment in the same indicated EGF profile before treatment) | | | | | | | |

Table 7 shows the measurement results of the endometrial EGF concentration, normalization rate, and conception rate in each treatment hroup. As a result, administration of rOPN and peptide 1 to a cow that exhibited an abnormal endometrial EGF concentration profile improved the abnormal expression of endometrial EGF and also improved the conception rate. In cows that originally had a normal endometrial EGF concentration profile, no changes in the conception rate due to rOPN or peptide 1 administration were observed. As a result, conception chance was improved in the rOPN and peptide 1 treatment groups compared to the untreated group.

## Claims

1. A ruminant conception chance-improving composition, which contains as an active ingredient a peptide or polypeptide containing the amino acid sequence represented by SEQ ID NO: 1 (SVAYGLK).

2. The conception chance-improving composition according to claim 1, wherein the peptide or polypeptide comprises an amino acid sequence represented by SEQ ID NO: 2 (GRGDSVAYGLK).

3. The conception chance-improving composition according to claims 1 or 2, wherein the peptide or polypeptide is a partial peptide of bovine osteopontin.

4. A conception chance-improving composition in a ruminant, wherein a bovine osteopontin protein, a mutant of a bovine osteopontin protein, or a partial peptide of these proteins is used as the active ingredient,
the respective protein, the respective mutant, and the respective partial peptide comprise an amino acid sequence represented by SEQ ID NO: 1 (SVAYGLK).

5. The conception chance-improving composition according to claim 4, wherein the respective protein, the respective mutant, and the respective partial peptide comprise an amino acid sequence represented by SEQ ID NO: 2 (GRGDSVAYGLK).

6. The conception chance-improving composition according to claim 4 or 5, wherein the amino acid sequence of a mutant of the protein of bovine osteopontin is an amino acid sequence having an amino acid sequence of 90% or more sequence identity with the protein of bovine osteopontin.

7. The conception chance-improving composition according to claim 4, wherein the peptide consisting of the amino acid sequence represented by SEQ ID NOs: 1 or 2 is the active ingredient.

8. The conception chance-improving composition according to any one of claims 1 to 7, wherein the respective ruminant is a ruminant after delivery.

9. The conception chance-improving composition according to any one of claims 1 to 8, wherein the respective ruminant is a cow, goat, sheep, deer, or giraffe.

10. The conception chance-improving composition according to Claim 8, wherein the respective ruminant is a cow 45-80 days after delivery.

11. The conception chance-improving composition according to any one of claims 1 to 9, wherein the respective ruminant is a cow with low conception chance.

12. The conception chance-improving composition according to Claim 11, wherein the cow with low conception chance is a cow whose increased epidermal growth factor concentration in the endometrium, which is observed in cows with normal conception chance on days 2-4 of estrus, has disappeared or is declining.

13. The conception chance-improving composition according to claim 11, wherein the low conception chance cow is a cow whose epidermal growth factor concentration in the endometrium on the third day from estrus is less than 4.7 ng / g per tissue weight.

14. The conception chance-improving composition according to claim 11, wherein the respective active ingredient comprises:
a protein comprising an amino acid sequence represented by SEQ ID NO: 1 (SVAYGLK) as a mutant of a protein of bovine osteopontin;
a partial peptide of a protein of bovine osteopontin comprising the amino acid sequence represented by SEQ ID NO: 1 (SVAYGLK), or
a partial peptide of a mutant of a protein of bovine osteopontin comprising an amino acid sequence represented by SEQ ID NO: 1 (SVAYGLK); and the cow with low conception chance is a repeat breeder cow.

15. The conception chance-improving composition according to any one of claims 1 to 14, wherein administration of the conception chance-improving composition normalizes the epidermal growth factor concentration profile in the endometrium from administration to one cycle of estrus.

16. A method of improving conception chance in a ruminant, wherein the conception chance-improving composition according to any one of claims 1 to 15 is administered to or in the vicinity of the vaginal fornix of the ruminant's vagina on the day of estrus and the period before and after the same.

17. The method of improving conception chance according to claim 16, wherein it is a solution in which the active ingredient is dissolved in 0.1 to 20 mL of the solution the solution of which can be administered to ruminants;
a solution that can be administered to the vagina, cervix, and uterus of ruminants; a solution that can be water, a buffer solution, the semen of the ruminant, or a solution containing appropriate and pharmaceutically acceptable additives that can be administered to the vagina, cervix, and uterus of the ruminant.

18. The method of improving conception chance according to claims 16 or 17, wherein the amount of the active ingredient contained in the conception chance-improving composition is 40 nmol or more.

19. The method of improving conception chance according to any one of claims 16-18 wherein the respective conception chance-improving composition is administered before, after, or simultaneously with artificial insemination.

20. The method of improving conception chance according to any one of claims 16 to 18, wherein the respective ruminant is a ruminant that naturally mates.

21. A method of improving conception chance according to any one of claims 16 to 18, wherein the respective ruminant is a ruminant to which a fertilized egg is implanted.

22. A method of increasing conception chance according to any one of claims 16 to 21, wherein the respective ruminant is a cow, goat, sheep, deer, or giraffe.

23. A straw for artificial insemination, wherein it is an artificial insemination straw filled with the conception chance-improving composition according to any one of claims 1 to 15,
wherein the active ingredient can be dissolved in 0.1 to 20 mL of the solution; the solution of which can be administered to ruminants;
a solution that can be administered to the vagina, cervix, and uterus of ruminants; a solution that can be water, a buffer solution, the semen of the ruminant, or a solution containing appropriate and pharmaceutically acceptable additives that can be administered to the vagina, cervix, and uterus of the ruminant.
